# EUROPEAN PATENT SPECIFICATION

(11) **EP 4 178 637 B1**
(45) Date of publication and mention of the grant of the patent: **14.05.2025**
(21) Application number: 21749508.4
(22) Date of filing: 13.07.2021
(51) Int. Cl.: A61L 29/04, A61L 29/08, A61L 29/14, A61L 29/16

(54) **MEDICAL DEVICES HAVING ANTI-MICROBIAL PROPERTIES AND METHODS FOR MAKING THE SAME**
MEDIZINISCHE VORRICHTUNGEN MIT ANTIMIKROBIELLEN EIGENSCHAFTEN UND VERFAHREN ZUR HERSTELLUNG DAVON
DISPOSITIFS MÉDICAUX AYANT DES PROPRIÉTÉS ANTIMICROBIENNES ET LEURS PROCÉDÉS DE FABRICATION

(30) Priority: 13.07.2020 US 202063050935 P
(43) Date of publication of application: 17.05.2023
(60) Divisional application: 25165417.4
(73) Proprietor: Hollister Incorporated, Libertyville IL 60048 (US)
(72) Inventor: MURNAGHAN, Kevin, Llbertyville, IL 60048 (US); FARRELL, David, J., Llbertyville, IL 60048 (US)
(74) Representative: FRKelly
(86) International application number: PCT/US2021/041470
(87) International publication number: WO 2022/015755

(56) References cited:
- US-A1- 2007 093 894
- NEOH KOON GEE ET AL: "Surface modification strategies for combating catheter-related complications: recent advances and challenges", JOURNAL OF MATERIALS CHEMISTRY. B, vol. 5, no. 11, 1 January 2017 (2017-01-01), GB, pages 2045 - 2067, XP055850895, ISSN: 2050-750X, Retrieved from the Internet <URL:https://pubs.rsc.org/en/content/articlepdf/2017/tb/c6tb03280j> DOI: 10.1039/C6TB03280J

## Description

Provisional Application No. 63/050,935, filed July 13, 2020.

### Technical Field

The present disclosure is directed to lubricious medical devices that include an anti-microbial disposed on an outer surface of the medical device to enhance the anti-microbial properties of the medical device. More specifically, the present disclosure is directed to medical devices that include a sulfanilamide, substituted sulfanilamide and/or a sulfanilamide derivative disposed on the outer surface of the medical device.

### Background

Insertable medical devices such as catheters are used to treat and facilitate treatment for a wide variety of medical conditions. These insertable medical devices have a myriad of uses including opening bodily passageways, delivering drugs, and aiding surgeons in surgeries. For example, urinary catheters are used to treat urinary incontinence. Intermittent urinary catheters are often used for self-catherization in non-sterile environments. Because these devices are inserted into the body, sterility and maintaining sterility is advantageous in preventing infections.

In such devices, pathogens including bacteria and microbes may be present on the surface or, where applicable, the coating of the medical devices. These pathogens can contaminate the medical device, causing the sterility of the catheter to be compromised. This contamination may result in catheter users, many of whom may be at an increased risk for infection, developing an infection. Thus, there remains a need for medical devices which resist pathogenic contamination.

US 2007/093894 A1 discloses a bacteriostatic and bactericidial antimicrobial composition to reduce putative colonization of a medical device by coating the medical device; and a method for coating a medical device comprising the steps of applying to at least a portion of the medical device, a bactericidal coating layer comprising a bactericidal agent; and applying to at least a portion of the surface of the medical device, a bacteriostatic coating comprising a bacteriostatic agent. The bactericidal agent can be a sulfonamide, such as sulfamethoxazole, sulfadiazine, sulfamethoxazole, sulfamethizole, sulfacarbamide, sulfadoxine. The antimicrobial agents can be applied to the medical device in a variety of methods, such as spraying, painting, dipping, sponging, atomizing, smearing, impregnating and spreading.

### Summary

The invention relates to a method of making a medical device having antimicrobial properties. The method includes reducing a diazonium sulfanilamide salt or a diazonium diaminodiphenyl sulfone salt in the presence of a reducing agent and a surface of the medical device whereby at least one of a sulfanilamide or a diaminodiphenyl sulfone attaches to the surface of the medical device.

### Brief Description of the Drawings

Fig. 1 is a perspective view of a medical device; and
Fig. 2 is a schematic view of a chemical process, showing formation of a sulfanilamide and the attachment of the sulfanilamide to a surface of the medical device of Fig. 1.

### Description of the Illustrated Embodiments

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

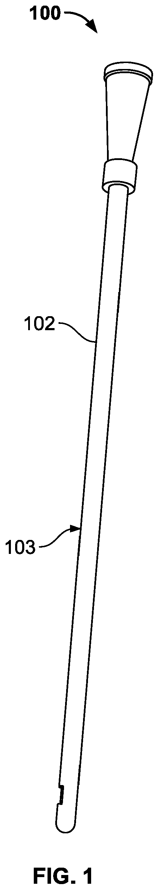

Fig. 1 shows a medical device 100, such as a urinary catheter. Although the subject matter disclosed herein may be described relative to urinary catheters, the subject matter is not limited to such, and such subject matter may apply to other suitable medical devices as well. The medical devices may be, for example, those which are configured for insertion into a lumen of a human body, such as the urethra, fallopian tubes, nasal passages or esophagus. Such medical devices may include, but are not limited to, catheters and endoscopes.

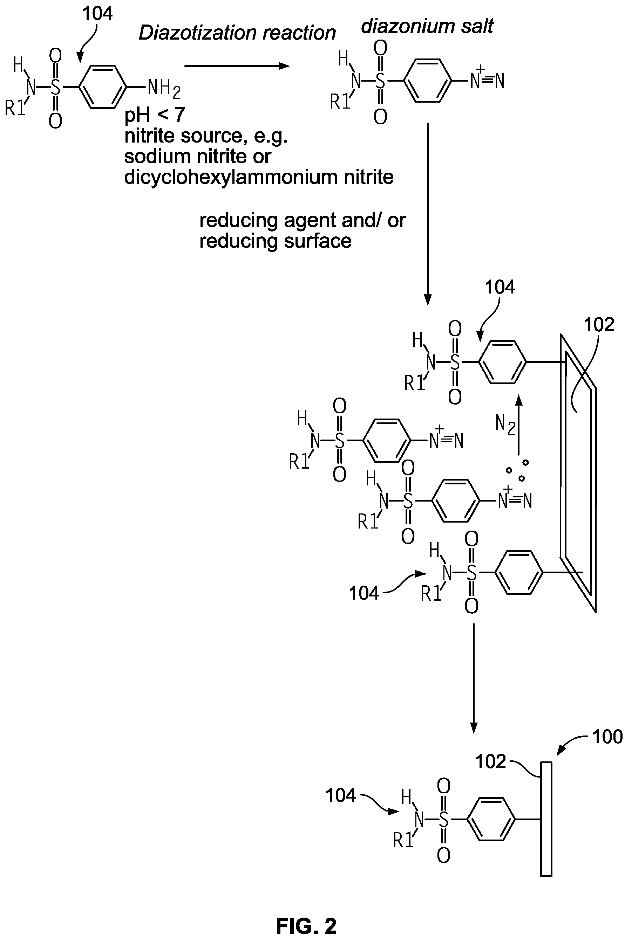

The medical device 100 comprises a surface 102. A sulfanilamide 104 (shown in Fig. 2) may be associated with the surface 102 and may be attached to the surface 102. In an embodiment, the sulfanilamide 104 may be attached to the surface 102 by grafting (i.e. chemical grafting). Grafting the sulfanilamide 104 to the surface 102 results in the surface having biocidal and antimicrobial properties. As used herein the term sulfanilamide(s) may encompass sulfanilamides, substituted sulfanilamides, and/or sulfanilamide derivatives.

Turning to Fig. 2, the sulfanilamide 104 may be attached to the surface 102 by reducing a diazonium sulfanilamide salt in the presence of a reducing agent and, also in the presence of the surface 102 of the medical device 100, thereby attaching the sulfanilamide 104 to the surface 102 of the medical device 100. For example, the surface of a medical device may be placed in contact with a solution containing the diazonium sulfanilamide salt, a reducing agent, and an acid. In one alternative, the acid may be a Bronsted acid, such acids may include nitric acid, phosphoric acid, hydrochloric acid, sulfuric acid, tetrafluoroboric acid or hexafluorophosphoric acid. Some acids may not be suitable for and would not be used in the process described herein. Such acids include hexafluorometallic acids such as hexafluorozironic or hexafluorotitanic acid. In one embodiment, the pH of the solution may be less than 7, and may be for example, between 2 and 4.

The surface 102 of the medical device 100 may be a plastic or polymer surface. The reducing agent may include a metal, such as steel or aluminum. Alternatively, the reducing agent may be metallic powders including, but not limited to, steel, aluminum, iron, or zinc powder. In another alternative the surface of the device itself could be or could include the reducing agent. For example, the surface of the device could be a metal surface that acts as a reducing agent, or the surface could be a polymer that includes a metal reducing agent incorporated therein. In another alternative, organic reducing agents, such as but not limited to: acetyl phenyl hydrazine, tannic acid, benzoin, ascorbic acid (vitamin A), ascorbic acid 6-palmitate, alpha-tocopherol, gamma -tocopherol, 2,2,5,7,8-pentamethylchromanol, gallic acid [3,4,5-trihydroxybenzoic acid], pyrogallol [benzene-1 ,2,3-triol], hydroquinone and combinations thereof, may be used. The reducing agent may be formed in solution.

To form the diazonium sulfanilamide salt, the sulfanilamide 104 may undergo a diazotization reaction, as shown in the first reaction in the schematic of Fig. 2. The sulfanilamide 104 may be reacted with a nitrogen source, such as a nitrite, which may include but is not limited to sodium nitrite, and dicyclohexylammonium nitrite. The nitrogen source also may be nitrous acid, or other group I or group II salts of nitrous acid, such as potassium nitrite, or calcium nitrite. Additionally, any other suitable nitrogen source may be used. In one embodiment, the sulfanilamide and nitrogen source may be placed in a solution to form the diazonium salt.

The diazonium salt may be formed from a sulfanilamide such as: 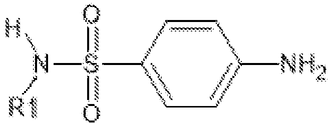 wherein R1 comprises one of (i) hydrogen, (ii) acetyl groups, (iii) amido groups, (iv) guanidyl groups, (v) 5 or 6 membered heterocyclic aromatic groups, (vii) methoxy-substituted heteroaromatic compounds and (vi) alkyl-substituted heteroaromatic compounds.

The 5 or 6 membered heterocyclic aromatic groups contain one or more of nitrogen atom(s), oxygen atom(s) or sulfur atom(s).

For example, the sulfanilamide 104 may be one or more of the below:

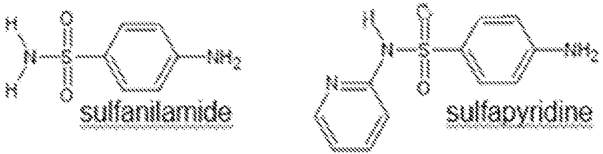

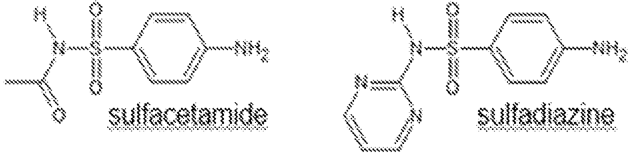

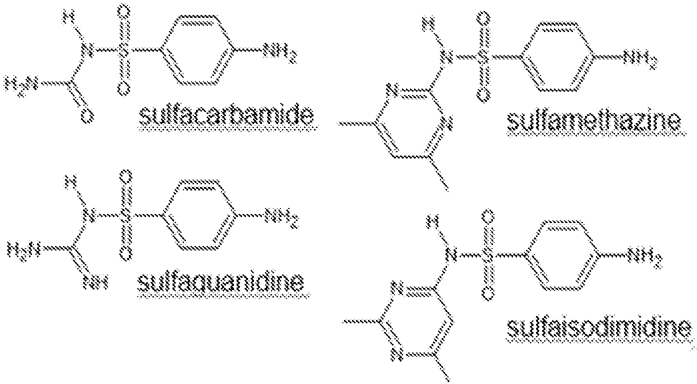

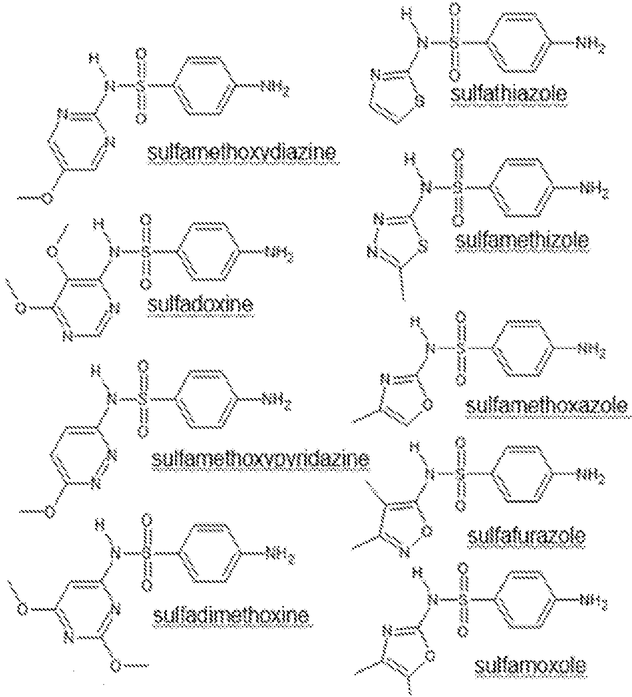

Other known suitable sulfanilamides besides those listed above may also be used. One of ordinary skill could readily understand that the above listed sulfanilamide and substituted sulfanilamides ("sulfanilamides") also represent corresponding derivatives of sulfanilamides. When attached to the medical device, the corresponding derivatives of the sulfanilamides have substantially the same structure as the above sulfanilamides except that the primary amine group of the sulfanilamides has been removed by reduction to facilitate attachment to the surface of the medical device. Reduction results in a covalently bound sulfanilamide attached to the surface of the medical device.

In an alternative to sulfanilamides, or in addition to, diaminodiphenyl sulfone (Dapsone) may be attached to the surface of a medical device using the same or similar mechanisms as disclosed herein. Diaminodiphenyl sulfone includes derivatives thereof, such as the diazonium forms of diaminodiphenyl sulfone. For example, diaminodiphenyl sulfone may be transformed into its mono or bis diazonium salt forms by the same or similar mechanisms as disclosed herein. The diazonium diaminodiphenyl sulfone salt may then be reduced in the presence of a medical device surface to attach diaminodiphenyl sulfone.

As shown in Fig. 2, reduction of the diazonium salt and the surface grafting process may result in stoichiometric amounts of nitrogen gas being released. The organic groups may also be bound to each other during the reduction process (coupling). Diazonium compounds may also be usable as coupling agents for further surface modification.

Thus, upon grafting, the sulfanamide 104 attached to the surface of the medical device may be any of the above mentioned sulfanilamides. In particular, the sulfanilamide may be: 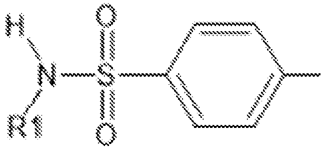 wherein R1 is any of the above-mentioned components or functional groups.

In an embodiment, the grafting of the sulfanilamide 104 to the surface 102 of the medical device 100 may result in a monolayer--a thin layer of organic groups grafted to the surface 102. In alternative embodiments, a plurality of layers may be grafted onto the medical device, for example a bilayer may be formed on the surface, resulting in a thicker layer of organic groups grafted onto the surface.

The medical device 100 may be a catheter. As illustrated in Fig. 1, the medical device 100 may be a urinary catheter. The catheter, such as the illustrated urinary catheter, may include a shaft 103 and the sulfanilamide 104 may be attached to the shaft 103. In an embodiment, the medical device surface may be bare plastic and the sulfanilamide may be grafted onto the plastic. The medical device 100 may be made of any appropriate material known to one of ordinary skill in the art including, but not limited to, low-density polyethylene (LDPE), TPE, PU, or other polymeric materials.

In alternative embodiments, the medical device may have a hydrophilic coating on a surface of the medical device. For example, a urinary catheter may have a hydrophilic coating disposed on the outer surface of the catheter. The sulfanilamide may be attached to the hydrophilic coating. For example, a lubricious hydrophilic polymer coating may be on the surface of the medical device. For example, the hydrophilic coating may be formed from polyvinylpyrrolidone or polyethylene oxide, which may optionally include agents and additives. In such an embodiment, the sulfanilamide may be grafted onto the hydrophilic coating. When the hydrophilic coating is contacted, wetted or hydrated with a hydration medium, it becomes lubricious. The lubricity eases introduction of the medical device into the body and aids in reducing pain and discomfort associated with such introduction. The hydrophilic coating can be a single layer or a multilayer hydrophilic coating. Multi-layered coating can include at least a base coat and a top layer. In other alternatives, coatings other than hydrophilic coatings may be on a surface of the medical device, wherein the sulfanilamide is attached to the coating.

The sulfanilamides described above are anti-bacterial, and anti-microbial. The sulfanilamides may be used to resist pathogens that infect the urinary tract and other areas of the body. For example, both sulfamethoxazole in combination with trimethoprim, and sulfadiazine may be useful in the treatment of urinary tract infections (UTIs). Sulfacetamide and its sodium salt are used in treatments for bacterial eye infections and also as a lotion used for the treatment of acne and dermatitis. Sulfaguanidine is a veterinary anti-bacterial used for intestinal infections. Thus, adding sulfanilamides to a surface of the medical device and/or to the hydrophilic coating may help prevent contamination of the medical device and/or the coating by inhibiting the proliferation of pathogens such as bacteria, and microbes.

## Claims

1. A method of making a medical device having antimicrobial properties, the method comprising:
reducing a diazonium sulfanilamide salt or a diazonium diaminodiphenyl sulfone salt in the presence of a reducing agent and a surface of a medical device, thereby attaching at least one of a sulfanilamide or a diaminodiphenyl sulfone to the surface of the medical device.

2. The method of claim 1, further including forming a diazonium sulfanilamide salt from a sulfanilamide or forming a diazonium diaminodiphenyl sulfone salt from a diaminodiphenyl sulfone.

3. The method of claim 2, wherein the diazonium sulfanilamide salt or the diazonium diaminodiphenyl sulfone salt is formed by a diazotization reaction.

4. The method of any one of claims 2 and 3, wherein forming the diazonium sulfanilamide salt or diazonium diaminodiphenyl sulfone salt comprises reacting the sulfanilamide or the diaminodiphenyl sulfone with a nitrogen source, preferably sodium nitrite or dicyclohexylammonium nitrite.

5. The method of any one of claims 2-4, wherein the sulfanilamide comprises: 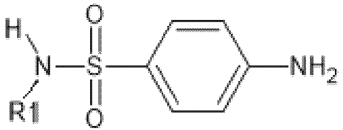 wherein R1 comprises one of hydrogen, acetyl groups, amido groups, guanidyl groups, 5 or 6 membered heterocyclic aromatic groups, methoxy-substituted heteroaromatic compounds and alkyl-substituted heteroaromatic compounds.

6. The method of claim 5, wherein the 5 or 6 membered heterocyclic aromatic groups contains one or more of nitrogen atom(s), oxygen atom(s) or sulfur atoms.

7. The method of any one of claims 2-6, wherein the sulfanilamide is one or more of: sulfanilamide, sulfapyridine, sulfamethoxydiazene, sulfathiazole, sulfacetamide, sulfadiazine, sulfadoxine, sulfamethizole, sulfacarbamide, sulfamethazine, sulfamethoxypyridazine, sulfamethoxazole, sulfafurazole, sulfaguanidine, sulfaisodimidine, sulfamethoxine, and sulfamoxole.

8. The method of any one of claims 1-7, wherein at least one of the sulfanilamide or the diaminodiphenyl sulfone is attached to the surface of the medical device by grafting.

9. The method of any one of claims 1-8, wherein the reducing agent comprises a metal, the metal comprising at least one of steel and aluminum.

10. The method of any one of claims 1-8, wherein the reducing agent comprises at least one of: iron powder, zinc powder, acetyl phenyl hydrazine, tannic acid, benzoin, ascorbic acid (vitamin A), ascorbic acid 6-palmitate, alpha-tocopherol, gamma - tocopherol, 2,2,5,7,8-pentamethylchromanol, gallic acid [3,4,5-trihydroxybenzoic acid], pyrogallol [benzene-1 ,2,3-triol], hydroquinone and combinations thereof.

11. The method of any one of claims 1-10, wherein the sulfanilamide comprises: 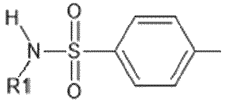 wherein R1 comprises one of hydrogen, acetyl groups, amido groups, guanidyl groups, 5 or 6 membered heterocyclic aromatic groups, methoxy-substituted heteroaromatic compounds and alkyl-substituted heteroaromatic compounds.

12. The method of claim 11, wherein the 5 or 6 membered heterocyclic aromatic groups contains one or more of nitrogen atom(s), oxygen atom(s) or sulfur atoms.

13. The method of any one of claims 1-12, wherein the sulfanilamide is one or more of: sulfanilamide, sulfapyridine, sulfamethoxydiazene, sulfathiazole, sulfacetamide, sulfadiazine, sulfadoxine, sulfamethizole, sulfacarbamide, sulfamethazine, sulfamethoxypyridazine, sulfamethoxazole, sulfafurazole, sulfaguanidine, sulfaisodimidine, sulfamethoxine, and sulfamoxole.

14. The method of any one of claims 1-13, wherein the medical device is a catheter having a shaft.

15. The method of claim 14, wherein at least one of the sulfanilamide or the diaminodiphenyl sulfone is attached to the shaft.

16. The method of any one of claims 1-14, wherein the medical device has a hydrophilic coating, wherein at least one of the sulfanilamide or the diaminodiphenyl sulfone is attached to the hydrophilic coating.

## Patentansprüche

1. Verfahren zum Herstellen einer medizinischen Vorrichtung mit antimikrobiellen Eigenschaften, wobei das Verfahren Folgendes umfasst:
Reduzieren eines Diazoniumsulfanilamidsalzes oder eines Diazoniumdiaminodiphenylsulfonsalzes in Gegenwart eines Reduktionsmittels und einer Oberfläche einer medizinischen Vorrichtung, wodurch mindestens eines von einem Sulfanilamid oder einem Diaminodiphenylsulfon an die Oberfläche der medizinischen Vorrichtung gebunden wird.

2. Verfahren nach Anspruch 1, ferner beinhaltend Bilden eines Diazoniumsulfanilamidsalzes aus einem Sulfanilamid oder Bilden eines Diazoniumdiaminophenylsulfonsalzes aus einem Diaminodiphenylsulfon.

3. Verfahren nach Anspruch 2, wobei das Diazoniumsulfanilamidsalz oder das Diazoniumdiaminodiphenylsulfonsalz durch eine Diazotierungsreaktion gebildet wird.

4. Verfahren nach einem der Ansprüche 2 und 3, wobei das Bilden des Diazoniumsulfanilamidsalzes oder Diazoniumdiaminodiphenylsulfonsalzes Umsetzen des Sulfanilamids oder des Diaminodiphenylsulfons mit einer Stickstoffquelle, vorzugsweise Natriumnitrit oder Dicyclohexylammoniumnitrit, umfasst.

5. Verfahren nach einem der Ansprüche 2-4, wobei das Sulfanilamid Folgendes umfasst: 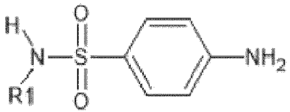 wobei R1 eines von Wasserstoff, Acetylgruppen, Amidogruppen, Guanidylgruppen, 5- oder 6-gliedrigen heterocyclischen aromatischen Gruppen, methoxysubstituierten heteroaromatischen Verbindungen und alkylsubstituierten heteroaromatischen Verbindungen umfasst.

6. Verfahren nach Anspruch 5, wobei die 5- oder 6-gliedrigen heterocyclischen aromatischen Gruppen eines oder mehrere von Stickstoffatom(en), Sauerstoffatom(en) oder Schwefelatomen enthalten.

7. Verfahren nach einem der Ansprüche 2-6, wobei es sich bei dem Sulfanilamid um eines oder mehrere von Folgenden handelt: Sulfanilamid, Sulfapyridin, Sulfamethoxydiazen, Sulfathiazol, Sulfacetamid, Sulfadiazin, Sulfadoxin, Sulfamethizol, Sulfacarbamid, Sulfamethazin, Sulfamethoxypyridazin, Sulfamethoxazol, Sulfafurazol, Sulfaguanidin, Sulfaisodimidin, Sulfamethoxin und Sulfamoxol.

8. Verfahren nach einem der Ansprüche 1-7, wobei mindestens eines von dem Sulfanilamid oder dem Diaminodiphenylsulfon durch Pfropfen an die Oberfläche der medizinischen Vorrichtung gebunden wird.

9. Verfahren nach einem der Ansprüche 1-8, wobei das Reduktionsmittel ein Metall umfasst, wobei das Metall mindestens eines von Stahl und Aluminium umfasst.

10. Verfahren nach einem der Ansprüche 1-8, wobei das Reduktionsmittel mindestens eines von Folgenden umfasst: Eisenpulver, Zinkpulver, Acetylphenylhydrazin, Gerbsäure, Benzoin, Ascorbinsäure (Vitamin A), Ascorbinsäure-6-palmitat, alpha-Tocopherol, gamma-Tocopherol, 2,2,5,7,8-Pentamethylchromanol, Gallussäure [3,4,5-Trihydroxybenzoesäure], Pyrogallol [Benzol-1,2,3-triol], Hydrochinon und Kombinationen davon.

11. Verfahren nach einem der Ansprüche 1-10, wobei das Sulfanilamid Folgendes umfasst: 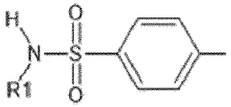 wobei R1 eines von Wasserstoff, Acetylgruppen, Amidogruppen, Guanidylgruppen, 5- oder 6-gliedrigen heterocyclischen aromatischen Gruppen, methoxysubstituierten heteroaromatischen Verbindungen und alkylsubstituierten heteroaromatischen Verbindungen umfasst.

12. Verfahren nach Anspruch 11, wobei die 5- oder 6-gliedrigen heterocyclischen aromatischen Gruppen eines oder mehrere von Stickstoffatom(en), Sauerstoffatom(en) oder Schwefelatomen enthalten.

13. Verfahren nach einem der Ansprüche 1-12, wobei es sich bei dem Sulfanilamid um eines oder mehrere von Folgenden handelt: Sulfanilamid, Sulfapyridin, Sulfamethoxydiazen, Sulfathiazol, Sulfacetamid, Sulfadiazin, Sulfadoxin, Sulfamethizol, Sulfacarbamid, Sulfamethazin, Sulfamethoxypyridazin, Sulfamethoxazol, Sulfafurazol, Sulfaguanidin, Sulfaisodimidin, Sulfamethoxin und Sulfamoxol.

14. Verfahren nach einem der Ansprüche 1 bis 13, wobei die medizinische Vorrichtung ein Katheter mit einem Schaft ist.

15. Verfahren nach Anspruch 14, wobei mindestens eines von dem Sulfanilamid oder dem Diaminodiphenylsulfon an den Schaft gebunden wird.

16. Verfahren nach einem der Ansprüche 1 bis 14, wobei die medizinische Vorrichtung eine hydrophile Beschichtung aufweist, wobei mindestens eines von dem Sulfanilamid oder dem Diaminodiphenylsulfon an die hydrophile Beschichtung gebunden ist.

## Revendications

1. Procédé de fabrication d'un dispositif médical ayant des propriétés antimicrobiennes, le procédé comprenant :
la réduction d'un sel de sulfanilamide diazonium ou d'un sel de diaminodiphénylsulfone diazonium en présence d'un agent réducteur et d'une surface d'un dispositif médical, fixant ainsi au moins l'un d'un sulfanilamide ou d'une diaminodiphénylsulfone à la surface du dispositif médical.

2. Procédé selon la revendication 1, comprenant également la formation d'un sel de sulfanilamide diazonium à partir d'un sulfanilamide ou la formation d'un sel de diaminodiphénylsulfone diazonium à partir d'une diaminodiphénylsulfone.

3. Procédé selon la revendication 2, dans lequel le sel de diazonium sulfanilamide ou le sel de diaminodiphénylsulfone diazonium est formé par une réaction de diazotation.

4. Procédé selon l'une quelconque des revendications 2 et 3, dans lequel la formation du sel de sulfanilamide diazonium ou du sel de diaminodiphénylsulfone diazonium comprend la réaction du sulfanilamide ou de la diaminodiphénylsulfone avec une source d'azote, de préférence du nitrite de sodium ou du nitrite de dicyclohexylammonium.

5. Procédé selon l'une quelconque des revendications 2 à 4, dans lequel le sulfanilamide comprend : 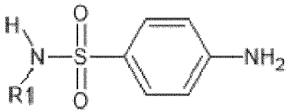 dans lequel R1 comprend l'un parmi l'hydrogène, les groupes acétyle, les groupes amido, les groupes guanidyle, les groupes aromatiques hétérocycliques à 5 ou 6 chaînons, les composés hétéroaromatiques substitués par méthoxy et les composés hétéroaromatiques substitués par alkyle.

6. Procédé selon la revendication 5, dans lequel les groupes aromatiques hétérocycliques à 5 ou 6 chaînons contiennent l'un ou plusieurs parmi un ou des atomes d'azote, un ou des atomes d'oxygène ou des atomes de soufre.

7. Procédé selon l'une quelconque des revendications 2 à 6, dans lequel le sulfanilamide est l'un ou plusieurs : du sulfanilamide, de la sulfapyridine, du sulfaméthoxydiazène, du sulfathiazole, du sulfacétamide, de la sulfadiazine, de la sulfadoxine, du sulfaméthizole, du sulfacarbamide, de la sulfaméthazine, de la sulfaméthoxypyridazine, du sulfaméthoxazole, du sulfafurazole, de la sulfaguanidine, de la sulfaisodimidine, de la sulfaméthoxine et du sulfamoxole.

8. Procédé selon l'une quelconque des revendications 1 à 7, dans lequel au moins l'un du sulfanilamide ou de la diaminodiphénylsulfone est fixé à la surface du dispositif médical par greffage.

9. Procédé selon l'une quelconque des revendications 1 à 8, dans lequel l'agent réducteur comprend un métal, le métal comprenant au moins l'un de l'acier et de l'aluminium.

10. Procédé selon l'une quelconque des revendications 1 à 8, dans lequel l'agent réducteur comprend au moins l'un : de la poudre de fer, de la poudre de zinc, de l'acétylphénylhydrazine, de l'acide tannique, de la benzoïne, de l'acide ascorbique (vitamine A), du 6-palmitate d'acide ascorbique, de l'alpha-tocophérol, du gamma-tocophérol, du 2,2,5,7,8-pentaméthylchromanol, de l'acide gallique [acide 3,4,5-trihydroxybenzoïque], du pyrogallol [benzène-1,2,3-triol], de l'hydroquinone et des combinaisons de ceux-ci.

11. Procédé selon l'une quelconque des revendications 1 à 10, dans lequel le sulfanilamide comprend : 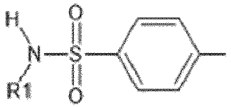 dans lequel R1 comprend l'un parmi l'hydrogène, les groupes acétyle, les groupes amido, les groupes guanidyle, les groupes aromatiques hétérocycliques à 5 ou 6 chaînons, les composés hétéroaromatiques substitués par méthoxy et les composés hétéroaromatiques substitués par alkyle.

12. Procédé selon la revendication 11, dans lequel les groupes aromatiques hétérocycliques à 5 ou 6 chaînons contiennent l'un ou plusieurs parmi un ou des atomes d'azote, un ou des atomes d'oxygène ou des atomes de soufre.

13. Procédé selon l'une quelconque des revendications 1 à 12, dans lequel le sulfanilamide est l'un ou plusieurs : du sulfanilamide, de la sulfapyridine, du sulfaméthoxydiazène, du sulfathiazole, du sulfacétamide, de la sulfadiazine, de la sulfadoxine, du sulfaméthizole, du sulfacarbamide, de la sulfaméthazine, de la sulfaméthoxypyridazine, du sulfaméthoxazole, du sulfafurazole, de la sulfaguanidine, de la sulfaisodimidine, de la sulfaméthoxine et du sulfamoxole.

14. Procédé selon l'une quelconque des revendications 1 à 13, dans lequel le dispositif médical est un cathéter présentant une tige.

15. Procédé selon la revendication 14, dans lequel au moins l'un du sulfanilamide ou de la diaminodiphénylsulfone est fixé à la tige.

16. Procédé selon l'une quelconque des revendications 1 à 14, dans lequel le dispositif médical présente un revêtement hydrophile, dans lequel au moins l'un du sulfanilamide ou de la diaminodiphénylsulfone est fixé au revêtement hydrophile.
